# NEUE EUROPÄISCHE PATENTSCHRIFT

(11) **EP 2 095 980 B2**
(45) Veröffentlichungstag und Bekanntmachung des Hinweises auf die Entscheidung über den Einspruch: **17.02.2021**
(45) Hinweis auf die Patenterteilung: 06.11.2013
(21) Anmeldenummer: 09002431.6
(22) Anmeldetag: 20.02.2009
(51) Int. Cl.: B60H 3/00, A61L 9/12

(54) **Vorrichtung zur Zuführung von Geruchsstoffen**
Device for supplying odorous substances
Dispositif d'introduction de matières odorantes

(30) Priorität: 27.02.2008 DE 102008011360
(43) Veröffentlichungstag der Anmeldung: 02.09.2009
(73) Patentinhaber: MAHLE Behr GmbH & Co. KG, 70469 Stuttgart (DE)
(72) Erfinder: Rais, Thomas, 71672 Marbach/Neckar (DE)
(74) Vertreter: Grauel, Andreas

(56) Entgegenhaltungen:
- WO-A-2004/037304
- JP-A- H0 584 284
- JP-A- H04 288 164
- US-A1- 2002 090 318
- US-A1- 2005 271 371

## Beschreibung

Die Erfindung betrifft eine Vorrichtung zur Zuführung wenigstens eines Geruchsstoffes in den Innenraum eines Kraftfahrzeuges, umfassend einen Geruchsstoffträger mit wenigstens einem Geruchsstoff, einen Luftkanal zur Zuführung von anzureichemder Luft zu dem Geruchsstoffträger und/oder zur Rückführung angereicherter Luft von dem Geruchsstoffträger in den Innenraum des Kraftfahrzeuges, wobei der wenigstens eine Geruchsstoff dem Luftkanal zuführbar ist, ein Gebläse zum Umwälzen der Luft in dem Luftkanal und eine Verschlusseinrichtung zur Steuerung und/oder Regelung der Menge des von dem Geruchsstoffträger in Abhängigkeit von der Zeit zuführbaren wenigstens einen Geruchsstoffes. Ferner betrifft die Erfindung ein Verfahren zur Zuführung wenigstens eines Geruchsstoffes in den Innenraum eines Kraftfahrzeuges mit den Schritten Leiten von Luft durch einen Luftkanal, Zuführen wenigstens eines Geruchsstoffes in die durch den Luftkanal geleitete Luft, Steuern und/oder Regeln der Menge des zugeführten wenigstens einen Geruchsstoffes in Abhängigkeit von der Zeit und Einleiten der mit dem wenigstens einen Geruchsstoff angereicherten Luft in den Innenraum des Kraftfahrzeuges.

Die Zuführung von Geruchsstoffen in die Atemluft kann beim Menschen verschiedene Reaktionen hervorrufen, z. B. positiv oder negativ auf seine Stimmungslage einwirken oder das subjektive Temperaturbefinden beeinflussen. Aus diesem Grund werden auch in Kraftfahrzeugen Vorrichtungen zur Zuführung wenigstens eines Geruchsstoffes in den Innenraum eingesetzt.

Aus der DE 103 28 747 A1 ist eine Klimaanlage mit einer Beduftungs-Vorrichtung und einer Temperatur-Einstellmöglichkeit bekannt, wobei eine Einstellvorrichtung für die Beduftungs-Vorrichtung zur Einstellung einer Duftnote vorgesehen ist.

Die DE 102 49 510 A1 zeigt eine gattungsbildende Vorrichtung zur Zuführung wenigstens eines Geruchsstoffes in den Innenraum eines Kraftfahrzeuges. Die Zufuhr von Geruchsstoffen in den Innenraum ist mittels einer Steuereinheit so einstellbar, dass ein aktueller Wert einer Geruchsstoffkonzentration im Innenraum im Bereich zwischen einer Wahmehmungsschwelle und einer Erkennungsschwelle liegt, wobei der Stoffübergang vom Geruchsstoff an einen Luftstrom für den Innenraum durch Verdunstung erfolgt. Hierzu wird mittels eines Gebläses Luft aus dem Innenraum angesaugt, durch einen Luftkanal geleitet und anschließend wieder in den Innenraum zurückgeführt. In den Luftkanal gelangen aus Duftkammem als Duftträgem die Geruchsstoffe, so dass der Innenraum mit den Geruchsstoffen angereichert wird. Mittels einer Verschlussvorrichtung kann die Duftkammer geöffnet und geschlossen, d. h. getaktet, werden, so dass die Menge des abgegebenen Geruchsstoffes in Abhängigkeit von der Zeit steuerbar ist. Die Taktung wird durch die Steuereinheit in Abhängigkeit von der aktuellen Temperatur im Fahrzeuginnenraum und der an der Klimaanlage eingestellten SollTemperatur ausgeführt.

Die US 2002/0090318 A1 zeigt eine Vorrichtung gemäß Oberbegriff des Anspruchs 1.

Nachteiligerweise kann die Temperatur der in den Luftkanal eingesaugten Luft von der Innenraumtemperatur abweichen, weil in dem Innenraum des Kraftfahrzeuges lokal erheblich unterschiedliche Temperaturen auftreten können. Wird beispielsweise die Luft aus der Mittelkonsole bzw. Instrumententafel angesaugt, kann durch die unterschiedliche Temperatur der umschließenden Bauteile, z. B. verursacht durch Sonneneinstrahlung oder durch die Abwärme eingebauter Geräte, beispielsweise Bildschirm, Radio oder CD-Spieler, die Temperatur an der Ansaugstelle für die Vorrichtung zur Zuführung eines Geruchsstoffes von der gemessenen Innenraumtemperatur abweichen. Die Sensoren zur Messung der Innenraumtemperatur sind im Allgemeinen an anderen Orten als die Ansaugstelle für den Luftkanal angeordnet. Die Steuerung der Taktung in Abhängigkeit von der Innenraumtemperatur berücksichtigt damit nicht die tatsächliche Temperatur der anzureichernden Luft in dem Luftkanal, so dass die Steuerung der Taktung fehlerbehaftet ist.

Die Aufgabe der vorliegenden Erfindung besteht deshalb darin, einer Vorrichtung und eine Verfahren zur Zuführung wenigstens eines Geruchsstoffes in den Innenraum eines Kraftfahrzeuges zur Verfügung zu stellen, bei der die Menge des abgegebenen Geruchsstoffes in Abhängigkeit von der Zeit als Funktion der tatsächlichen Temperatur der an dem Geruchsstoffträger vorbeigeleiteten Luft gesteuert wird.

Die Aufgabe wird gelöst mit einer Vorrichtung gemäß Anspruch 1.

Damit kann die Temperatur der an dem Geruchsstoffträger vorbeigeleitete Luft gemessen werden und mittels der Temperatursignale die Verschlusseinrichtung gesteuert und/oder geregelt werden. Dadurch können Fehler vermieden werden, welche aus einer Temperaturdifferenz zwischen der von einem Sensor gemessenen Innenraumtemperatur und der Temperatur der durch den Luftkanal geleiteten Luft resultieren. Die Vorrichtung kann in den Kanal einer Klimaanlage integriert werden, d. h. der Geruchsstoffträger wird von durch einen Wärmeaustauscher geleiteten Luft beaufschlagt und das Gebläse der Klimaanlage auch für die Vorrichtung genutzt. Außerdem kann das Gebläse der Klimaanlage für die Vorrichtung genutzt werden, jedoch mittels eine Bypasskanales die der Vorrichtung zugeführte Luft an dem Wärmetauscher vorbeigegleitet werden, so dass zu der Vorrichtung nicht gekühlte oder erwärmte Außenluft geleitet wird.

Erfindungsgemäß umfasst die Vorrichtung eine Steuereinheit und mittels in der Steuereinheit hinterlegter Kurven oder Algorithmen ist die Verschlusseinrichtung in Abhängigkeit von der Temperatur dahingehend steuerbar, dass in Abhängigkeit von der Zeit eine konstante oder variable Menge des wenigstens einen Geruchsstoffes zugeführt wird. Damit kann beispielsweise die Konzentration des wenigstens einen Geruchsstoffes taktweise zugegeben werden, so dass beim Benutzer kein Gewöhnungseffekt entsteht.

Insbesondere umfasst die Vorrichtung einen Sensor, insbesondere Oxidationssensor, zur Messung der Konzentration des wenigstens einen Geruchsstoffes in der angereicherten Luft und aufgrund der von dem Temperatursensor gemessenen Temperatur und der von dem Sensor gemessenen Konzentration ist mittels der Verschlusseiridchtung in Abhängigkeit von der Zeit eine im Wesentlichen konstante oder variable Menge des zuführbaren wenigstens einen Geruchsstoffes regelbar.

In einer weiteren Ausgestaltung ist unterhalb einer unteren Temperatur der durch den Luftkanal geleiteten Luft, z. B. -20° C, und/oder oberhalb einer oberen Temperatur der durch den Luftkanal geleiteten Luft, z. B. +60° C, das Gebläse ausgeschaltet und/oder verhindert die Verschlusseinrichtung die Zuführung des wenigstens einen Geruchsstoffes. Die Verwendung der von dem Temperatursensor gemessenen Temperatur für eine temperaturabhängige Betriebsstrategie dient dazu, Schäden an der Vorrichtung bei extremen Temperaturen zu verhindern.

Insbesondere ist von dem Gebläse die Luft aus dem Innenraum des Kraftfahrzeuges ansaugbar. Damit ist die Temperatur der zu dem Geruchsstoffträger geleiteten Luft wesentlich konstanter als wenn Luft aus der Klimaanlage entnommen wird, d. h. es entsteht ein im Wesentlicher konstanter Geruchsstoffeindruck. Die Luft aus der Klimaanlage ist im Sommer stark gekühlt und im Winter geheizt, so dass im Winter ein sehr starker und im Sommer ein sehr schwacher Geruchsstoffeindruck entstehen würde.

Erfindungsgemäß sind der Geruchsstoffträger und die Verschlusseinrichtung in dem Luftkanal angeordnet.

Zweckmäßig ist die Verschlusseinrichtung ein Schieber oder eine Klappe, welcher eine Öffnung zu dem Geruchsstoffträger abwechselnd vollständig öffnet oder schließt oder ein Schieber oder eine Klappe, welche die Öffnung zu dem Geruchsstoffträger variabel wenigstens teilweise öffnet bzw. schließt. Das vollständige Öffnen oder Schließen führt zu einer getakteten Zugabe des wenigstens einen Geruchsstoffes, so dass beim Benutzer kein Gewöhnungseffekt aufgrund einer ständig konstanten Konzentration des wenigstens einen Geruchsstoffes entsteht.

Ein erfindungsgemäßes Verfahren wird durch Anspruch 4 angeben.

In einer zusätzlichen Ausführungsform wird die Konzentration des wenigstens einen Geruchsstoffes in der angereicherten Luft in dem Luftkanal gemessen und in Abhängigkeit von der Temperatur der durch den Luftkanal geleiteten Luft und der Konzentration in Abhängigkeit von der Zeit die Menge des zugeführten wenigstens einen Geruchsstoffes geregelt.

Vorzugsweise wird oberhalb einer oberen Temperatur der durch den Luftkanal geleiteten Luft, z. B. +60° C, oder unterhalb einer unteren Temperatur der durch den Luftkanal geleiteten Luft, z. B. -20° C, keine Luft durch den Luftkanal geleitet und/oder das Zuführen des wenigstens einen Geruchsstoffes in die durch den Luftkanal geleitete Luft unterbunden.

In einer weiteren Ausführungsform wird oberhalb einer ersten obere Innenraumtemperatur, z. B. +45° C, oder unterhalb einer ersten unteren Innenraumtemperatur, z. B. 0° C, keine Luftdurch den Luftkanal geleitet und das Zuführen des wenigstens einen Geruchsstoffes in die Luft unterbunden, wobei die erste obere Innenraumtemperatur größer ist als die erste untere Innenraumtemperatur. Bei extremen Innenraumtemperaturen wird die Vorrichtung komplett abgeschaltet, um bei für den Benutzer unangenehmen Temperaturen keine Geruchsstoffe zuzuführen. Die Verwendung der Innenraumtemperatur für die Betriebsstrategie der Vorrichtung dient dazu, die Funktionsfähigkeit der Vorrichtung und für den Benutzer den Komfort zu erhöhen. Die Innenraumtemperatur wird von Sensoren im Innenraum gemessen.

In einer zusätzlichen Ausgestaltung wird oberhalb einer zweiten oberen Innenraumtemperatur, z. B. +35° C, oder unterhalb einer zweiten unteren Innenraumtemperatur, z. B. 10° C, Luft durch den Luftkanal geleitet und das Zuführen des wenigstens einen Geruchsstoffes in die Luft unterbunden, wobei die zweite obere Innenraumtemperatur größer ist als die zweite untere Innenraumtemperatur. Bei moderaten Temperaturen kann damit der Geruchsstoffträger vortemperiert werden.

Zweckmäßig ist die erste obere Innenraumtemperatur größer ist als die zweite obere Innenraumtemperatur und/oder die erste untere Innenraumtemperatur ist kleiner als die zweite untere Innenraumtemperatur.

In einer weiteren Ausführungsform wird in einem Teilbereich der Innenraumtemperatur zwischen der zweiten oberen Innenraumtemperatur und der zweiten unteren Innenraumtemperatur, z. B. zwischen 15° C und 35° C, Luft durch den Luftkanal geleitet und wenigstens ein Geruchsstoff der durch den Luftkanal geleitet Luft zugeführt. Bei angenehmen Innenraumtemperaturen wird die Vorrichtung damit komplett eingeschaltet.

Vorzugsweise wird mittels eines Gebläses die Luft durch den Luftkanal geleitet und/oder mittels eines Temperatursensors die Temperatur der durch den Luftkanal geleiteten Luft gemessen und/oder mittels eines Sensors, insbesondere Oxidationssensors, zur Messung der Konzentration des wenigstens einen Geruchsstoffes die Konzentration des wenigstens einen Geruchsstoffes in der mit dem wenigstens einen Geruchsstoff angereicherten Luft in dem Luftkanal gemessen.

Insbesondere wird die Luft aus dem Innenraum des Kraftfahrzeuges in den Luftkanal gesaugt.

Die Erfindung umfasst ferner ein Computerprogramm mit Programmcodemitteln, die auf einem computerlesbaren Datenträger gespeichert sind, um ein oben beschriebenes Verfahren durchzuführen, wenn das Computerprogramm auf einem Computer oder einer entsprechenden Recheneinheit durchgeführt wird.

Bestandteil der Erfindung ist außerdem ein Computerprogrammprodukt mit Programmcodemitteln, die auf einem computerlesbaren Datenträger gespeichert sind, um ein oben beschriebenes Verfahren durchzuführen, wenn das Computerprogramm auf einem Computer oder einer entsprechenden Recheneinheit durchgeführt wird.

Im Nachfolgenden werden vier Ausführungsbeispiele der Erfindung unter Bezugnahme auf die beigefügten Zeichnungen näher beschrieben. Es zeigt:
- Fig. 1: einen schematisierten Querschnitt einer Vorrichtung zur Zuführung wenigstens eines Geruchsstoffes in den Innenraum eines Kraftfahrzeuges in einem ersten Ausführungsbeispiel,
- Fig. 2: einen schematisierten Querschnitt der Vorrichtung gemäß Fig. 1 in einem zweiten Ausführungsbeispiel,
- Fig. 3: einen schematisierten Querschnitt der Vorrichtung zur Zuführung wenigstens eines Geruchsstoffes in den Innenraum des Kraftfahrzeuges in einem dritten Ausführungsbeispiel und
- Fig. 4: einen schematisierten Querschnitt der Vorrichtung gemäß Fig. 3 in einem vierten Ausführungsbeispiel.

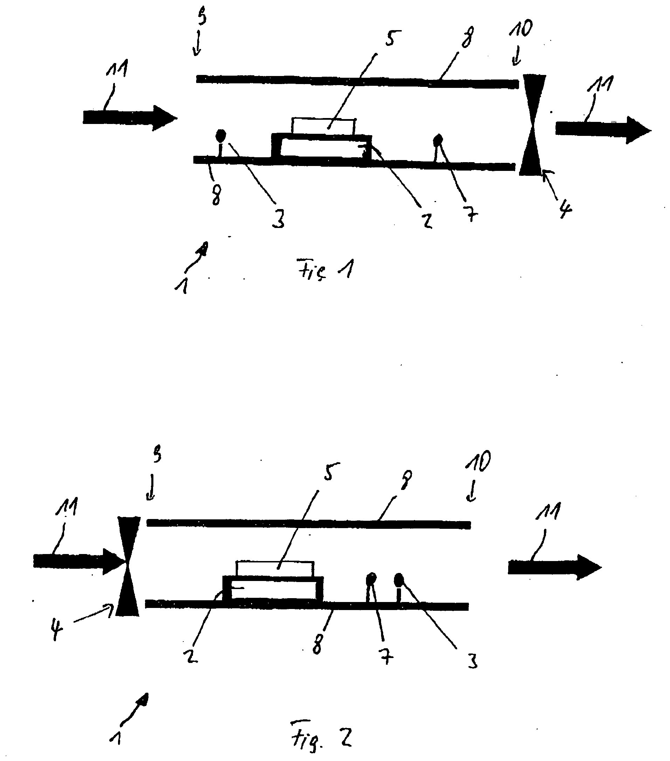

Fig. 1 zeigt schematisiert eine Vorrichtung 1 zur Zuführung wenigstens eines Geruchsstoffes in den Innenraum eines Kraftfahrzeuges (nicht dargestellt). Dadurch kann für die in dem Innenraum befindlichen Personen ein angenehmer Geruch erzeugt werden.

Die Vorrichtung 1 weist einen Luftkanal 8 auf. Der Luftkanal 8 verfügt über ein erstes Ende 9 und ein zweites Ende 10, welche jeweils in den Innenraum des Kraftfahrzeuges münden. Ein Gebläse 4 ist in dem Luftkanal 8 im Bereich des zweiten Endes 10 des Luftkanals 8 angeordnet. Mittels des Gebläses 4 wird an dem ersten Ende 9 des Luftkanals 9 Luft aus dem Innenraum angesaugt, durch den Luftkanal 9 geleitet und anschließend an dem zweiten Ende 10 des Luftkanals 8 wieder in den Innenraum zurückgeleitet. Der Luftstrom 11 ist durch Pfeile dargestellt. In dem Luftkanal 8 ist ein Geruchsstoffträger 2 positioniert. Der Geruchsstoffträger 2 gibt an die durch den Luftkanal 8 strömende Luft Geruchsstoffe ab, so dass mit Geruchsstoffen beaufschlagte bzw. angereicherte Luft in den Innenraum zurückgeführt wird. Zwischen dem ersten Ende 9 des Luftkanals 8 und dem Geruchsstoffträger 2 ist in dem Luftkanal 8 ein Temperatursensor 3 angeordnet. Der Temperatur sensor 3 misst die Temperatur der anzureichemden, durch den Luftkanal 8 strömenden Luft. Ferner ist in dem Luftkanal 8 zwischen dem Geruchsstoffträger 2 und dem zweiten Ende 10 des Luftkanals 8 ein optionaler Sensor 7 zur Messung der Konzentration der Geruchsstoffe in der durch den Luftkanal 8 strömenden Luft angeordnet.

Die von dem Temperatursensor 3 gelieferten Messwerte werden in einer Steuerungseinheit (nicht dargestellt), welche auch Bestandteil der Kraftfahrzeugelektronik sein kann, ausgewertet und in Abhängigkeit von den Messwerten wird die Menge der von dem Geruchsstoffträger 2 an die Luft in Abhängigkeit von der Zeit abgegebenen Geruchsstoffe gesteuert. Der Geruchsstoffträger 2 umfasst eine Geruchsstoffkammer, die in Verbindung mit einer austauschbaren und wiederbefüllbaren Kartusche steht (nicht dargestellt). Die Kartusche gibt die Geruchsstoffe an die Geruchsstoffkammer ab, indem die flüssigen Geruchsstoffe in der Kartusche verdampfen. Ein von der Geruchsstoffkammer in den Luftkanal mündender Auslass ist mit einer Verschlusseinrichtung 5 variable verschließbar, so dass die Menge der abgegebenen Geruchsstoffe steuerbar ist.

In der Steuerungseinheit sind entsprechend Funktion oder Kurven hinterlegt, welche die Stellung der Verschlusseinrichtung 5 in Abhängigkeit von der Temperatur angeben. Dadurch kann beispielsweise eine konstante Menge an abgegebenen Geruchsstoffen - unabhängig von der Temperatur der durch den Luftkanal 9 strömenden Luft - erreicht werden. Außerdem ist es möglich, die Menge an abgegebenen Geruchsstoffen variable in Abhängigkeit von der Temperatur zu steuern, um damit bestimmte Effekte zu erzielen. Beispielsweise kann die gleiche Konzentration an Geruchsstoffen bei unterschiedlichen Temperaturen bei dem Benutzer einen unterschiedlichen subjektiven Eindruck hinterlassen, so dass mittels einer entsprechend angepassten Menge an Geruchsstoffen subjektiv auch bei unterschiedlichen Temperaturen der gleiche Eindruck bei dem Benutzer entsteht. Der Benutzer hat außerdem die Möglichkeit, die abgegebene Menge an Geruchsstoffen zu steuern, d. h. er kann diese nach seinen Wünschen erhöhen, erniedrigen oder vollständig abschalter.

Der als Oxidationssensor ausgebildete Sensor 7 dient zur Messung der Konzentration der Geruchsstoffe in der angereicherten Luft und ermöglicht eine Regelung der Konzentration. Die Messwerte des Sensors 7 werden an die Steuerungseinheit übermittelt, so dass die Konzentration der Geruchsstoffe der angereicherten Luft in dem Luftkanal 9 messbar und damit regelbar ist.

Der Temperatursensor 3 ermöglicht außerdem auch eine temperaturabhängige Betriebsstrategie der Vorrichtung 1.

In einer ersten Betriebsstrategie wird unterhalb einer bestimmten Temperatur der durch den Luftkanal 8 geleiteten Luft, z. B. -20° C, oder oberhalb einer bestimmten Temperatur, z. B. +60° C, das Gebläse 4 abgeschaltet und mittels der Verschlusseinrichtung 5 das Zuführen der Geruchsstoffe in die Luft unterbunden. Diese Betriebsstrategie kann dadurch verfeinert werden, indem z. B. bei hohen Temperaturen in der Vorrichtung 1 das Gebläse 4 eingeschaltet wird, damit heiße Stauluft aus dem Luftkanal 8 geblasen wird und währenddessen der Austrag der Geruchsstoffe unterbunden bleibt. Bei tiefen Temperaturen in der Vorrichtung 1 kann analog das Gebläse 4 eingeschaltet werden, um die Vorrichtung 1 bei bereits erwärmter Innenraumluft ebenfalls zu erwärmen. Die Steuerungseinheit kann hierzu die Innenraumtemperatur mit der mit der Temperatur der Luft in dem Luftkanal 8 vergleichen.

Bei extremen Innenraumtemperaturen, z. B. weniger als 0° C oder mehr als 45° C, sind das Gebläse 4 auszuschalten und der Austrag der Geruchstoffe blockiert. Bei moderaten Innenraumtemperaturen, beispielsweise zwischen 10° C und 45° C, kann das Gebläse 4 eingeschaltet werden, um die Vorrichtung 1 vorzutemperieren, wobei die Abgabe der Geruchsstoffe unterbunden bleibt. Erst ab einer für die Abgabe der Geruchsstoffe geeigneten Innentemperaturbereich zwischen 15° C und 35° C der in dem Luftkanal 9 befindlichen Luft werden die Geruchsstoffe in den Luftkanal 9 abgegeben. Die Innenraumtemperatur wird mit Sensoren im Innenraum gemessen (nicht dargestellt).

In Fig. 2 ist ein zweites Ausführungsbeispiel der Vorrichtung 1 dargestellt. Abweichend von dem ersten Ausführungsbeispiel gemäß Fig. 1 ist das Gebläse 4 am ersten Ende 9 des Luftkanals 8 positioniert. Ansonsten entspricht das zweite Ausführungsbeispiel dem ersten Ausführungsbeispiel

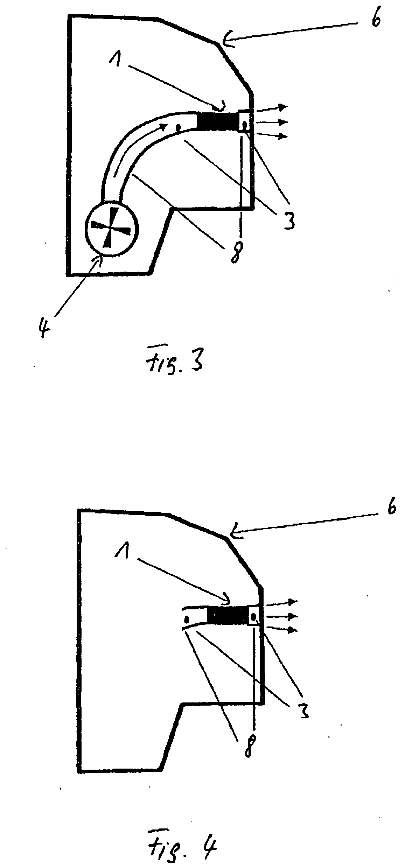

Fig. 3 zeigt ein drittes Ausführungsbeispiel der Vorrichtung 1. Die Vorrichtung 1 ist in eine Instrumententafel 6 des Kraftfahrzeuges eingebaut. Das Gebläse 4 ist ein externes Gebläse 4 einer Klimaanlage des Kraftfahrzeuges. Der Temperatursensor 3 ist in Strömungsrichtung der Luft in dem Luftkanal 8 entweder vor oder nach dem Geruchsstoffträger 2 angeordnet, wobei in Fig. 3 beide Varianten dargestellt sind. Der optionale Sensor 7 ist nicht dargestellt. Ansonsten entspricht das dritte Ausführungsbeispiel dem ersten Ausführungsbeispiel.

In Fig. 4 wird ein viertes Ausführungsbeispiel der Vorrichtung 1 offenbart. Die Vorrichtung 1 ist in eine Instrumententafel 6 des Kraftfahrzeuges eingebaut. Das Gebläse 4 ist in die Vorrichtung 1 integriert und nicht dargestellt. Der Temperatursensor 3 ist in Strömungsrichtung der Luft in dem Luftkanal 8 entweder vor oder nach dem Geruchsstoffträger 2 angeordnet, wobei in Fig. 4 beide Varianten dargestellt sind. Der optionale Sensor 7 ist nicht dargestellt. Ansonsten entspricht das vierte Ausführungsbeispiel dem ersten Ausführungsbeispiel.

Insgesamt betrachtet können mit der Vorrichtung 1 zur Zuführung von Geruchsstoffen in den Innenraum des Kraftfahrzeuges erhebliche Vorteile erreicht werden. Anstelle der Innenraumtemperatur wie im Stand der Technik wird zur Steuerung und/oder Regelung der Zugabe der Geruchsstoffe mittels eines in dem Luftkanal 8 angeordneten Temperatursensors 3 die Temperatur der durch die Vorrichtung 1 strömenden Luft gemessen. Diese Temperatur ist die maßgebliche Temperatur. Die Innenraumtemperatur wird von Sensoren gemessen, die im Allgemeinen an einem anderen Ort innerhalb des Innenraumes angeordnet sind als die Luft in den Luftkanal 9 angesaugt wird. Dadurch weicht häufig die von den Sensoren für die Innenraumtemperatur gemessene Temperatur von der Temperatur der von der Vorrichtung 1 angesaugten Luft ab. Die aus der Differenz zwischen der von den Sensoren für die Innenraumtemperatur gemessenen Temperatur und der Temperatur der von der Vorrichtung 1 angesaugten Luft resultierenden Fehler können dadurch in besonders vorteilhafter und einfacher Weise vermieden werden.

## Patentansprüche

1. Vorrichtung (1) zur Zuführung wenigstens eines Geruchsstoffes in den Innenraum eines Kraftfahrzeuges, umfassend
- einen Geruchsstoffträger (2) mit wenigstens einem Geruchsstoff,
- einen Luftkanal (8) zur Zuführung von anzureichernder Luft zu dem Geruchsstoffträger (2) und/oder zur Rückführung angereicherter Luft von dem Geruchsstoffträger (2) in den Innenraum des Kraftfahrzeuges, wobei der wenigstens eine Geruchsstoff dem Luftkanal (8) zuführbar ist,
- *ein Gebläse (4) zum Umwälzen der Luft in dem Luftkanal (8) und*
- eine Verschlusseinrichtung (5) zur Steuerung oder Regelung der Menge des von dem Geruchsstoffträger (2) in Abhängigkeit von der Zeit zuführbaren wenigstens einen Geruchsstoffes,
wobei mittels wenigstens eines Temperatursensors (3) die Temperatur der in dem Luftkanal (8) befindlichen mit dem wenigstens einen Geruchsstoff anzureichernden und/oder angereicherten Luft messbar ist und in Abhängigkeit von der gemessenen Temperatur die Verschlusseinrichtung (5) Steuer- oder regelbar ist, **dadurch gekennzeichnet, dass** der Geruchsstoffträger (2) im Luftkanal (8) angeordnet ist und der wenigstens eine Temperatursensor (3) im und/oder an dem Luftkanal (8) angeordnet ist, wobei die Vorrichtung (1) eine Steuereinheit umfasst und mittels in der Steuereinheit hinterlegter Kurven oder Algorithmen die Verschlusseinrichtung (5) in Abhängigkeit von der Temperatur dahingehend steuerbar ist, dass in Abhängigkeit von der Zeit eine konstante oder variable Menge des wenigstens einen Geruchsstoffes zugeführt wird.

2. Vorrichtung nach Anspruch 1, **dadurch gekennzeichnet, dass** unterhalb einer unteren Temperatur der durch den Luftkanal (8) geleiteten Luft, z. B. - 20° C, und/oder oberhalb einer oberen Temperatur der durch den Luftkanal geleiteten Luft, z. B. +60° C, das Gebläse (4) ausgeschaltet ist und/oder die Verschlusseinrichtung (5) die Zuführung des wenigstens einen Geruchsstoffes verhindert.

3. Vorrichtung nach Anspruch 1 oder 2, **dadurch gekennzeichnet, dass** die Verschlusseinrichtung (5) ein Schieber oder eine Klappe ist, welcher eine Öffnung zu dem Geruchsstoffträger (2) abwechselnd vollständig öffnet oder schließt oder ein Schieber oder eine Klappe ist, welche die Öffnung zu dem Geruchsstoffträger (2) variabel wenigstens teilweise öffnet bzw. schließt.

4. Verfahren zur Zuführung wenigstens eines Geruchsstoffes in den Innenraum eines Kraftfahrzeuges mit den Schritten
- Leiten von Luft durch einen Luftkanal (8),
- Zuführen wenigstens eines Geruchsstoffes in die durch den Luftkanal (8) geleitete Luft,
- Steuern oder Regeln der Menge des zugeführten wenigstens einen Geruchsstoffes in Abhängigkeit von der Zeit und
- Einleiten der mit dem wenigstens einen Geruchsstoff angereicherten Luft in den Innenraum des Kraftfahrzeuges,
wobei in Abhängigkeit von der Temperatur der durch den Luftkanal (8) geleiteten Luft die Menge des zugeführten wenigstens einen Geruchsstoffes gesteuert oder geregelt wird, **dadurch gekennzeichnet, dass** der Geruchsstoffträger (2) im Luftkanal (8) angeordnet ist und der wenigstens eine Temperatursensor (3) im und/oder an dem Luftkanal (8) angeordnet ist, wobei in Abhängigkeit von der Zeit die, vorzugsweise variable, Menge des zugeführten wenigstens einen Geruchsstoffes von in einer Steuereinheit hinterlegter Kurven oder Algorithmen in Abhängigkeit von der Temperatur dahingehend steuerbar ist, dass in Abhängigkeit von der Zeit eine konstante oder variable Menge des wenigstens einen Geruchsstoffes zugeführt wird.

5. Verfahren nach Anspruch 4, **dadurch gekennzeichnet, dass** oberhalb einer oberen Temperatur der durch den Luftkanal (8) geleiteten Luft, z. B. +60° C, oder unterhalb einer unteren Temperatur der durch den Luftkanal (8) geleiteten Luft, z. B. -20° C, keine Luft durch den Luftkanal (8) geleitet wird und/oder das Zuführen des wenigstens einen Geruchsstoffes in die durch den Luftkanal (8) geleitete Luft unterbunden wird.

6. Verfahren nach Anspruch 4 oder 5, **dadurch gekennzeichnet, dass** oberhalb einer ersten oberen Innenraumtemperatur, z. B. +45° C, oder unterhalb einer ersten unteren Innenraumtemperatur, z. B. 0° C, keine Luft durch den Luftkanal (8) geleitet wird und das Zuführen des wenigstens einen Geruchsstoffes in die Luft unterbunden wird, wobei die erste obere Innenraumtemperatur größer ist als die erste untere Innenraumtemperatur.

7. Verfahren nach Anspruch 6, **dadurch gekennzeichnet, dass** oberhalb einer zweiten oberen Innenraumtemperatur, z. B. +35° C, oder unterhalb einer zweiten unteren Innenraumtemperatur, z. B. 10° C, Luft durch den Luftkanal (8) geleitet wird und das Zuführen des wenigstens einen Geruchsstoffes in die Luft unterbunden wird, wobei die zweite obere Innenraumtemperatur größer ist als die zweite untere Innenraumtemperatur.

8. Verfahren nach Anspruch 7, **dadurch gekennzeichnet, dass** die erste obere Innenraumtemperatur größer ist als die zweite obere Innenraumtemperatur und/oder die erste untere Innenraumtemperatur kleiner ist die zweite untere Innenraumtemperatur.

9. Verfahren nach Anspruch 7 oder 8, **dadurch gekennzeichnet, dass** in einem Teilbereich der Innenraumtemperatur zwischen der zweiten oberen Innenraumtemperatur und der zweiten unteren Innenraumtemperatur, z. B. zwischen 15° C und 35° C, Luft durch den Luftkanal (8) geleitet wird und wenigstens ein Geruchsstoff der durch den Luftkanal (8) geleiteten Luft zugeführt wird.

10. Computerprogrammprodukt mit Programmcodemitteln, die auf einem computerlesbaren Datenträger gespeichert sind, um ein Verfahren nach einem oder mehreren der Ansprüche 4 bis 9 durchzuführen, wenn das Computerprogramm auf einem Computer oder einer entsprechenden Recheneinheit durchgeführt wird.

## Claims

1. A device (1) for supplying at least one odorous substance into the passenger compartment of a motor vehicle, comprising:
- an odorous substance carrier (2) with at least one odorous substance,
- an air duct (8) for supplying air to be enriched to the odorous substance carrier (2) and/or for recirculating enriched air from the odorous substance carrier (2) into the passenger compartment of the motor vehicle, wherein the at least one odorous substance can be supplied to the air duct (8),
- a fan (4) for circulating the air in the air duct (8), and
- a closing device (5) for controlling or regulating the amount of the at least one odorous substance that can be supplied from the odorous substance carrier (2) as a function of the time,
wherein the temperature of the air that is present in the air duct (8) and to be enriched and/or enriched with the at least one odorous substance can be measured by at least one temperature sensor (3), and the closing device (5) can be controlled or regulated as a function of the measured temperature, **characterised in that** the odorous substance carrier (2) is disposed in the air duct (8) and the at least one temperature sensor (3) is disposed in and/or on the air duct (8), wherein the device (1) comprises a control unit and the closing device (5) can be controlled by way of curves or algorithms stored in the control unit as a function of the temperature to the effect that a constant or variable amount of the at least one odorous substance is supplied as a function of the time.

2. The device according to claim 1, **characterised in that** the fan (4) is deactivated and/or the closing device (5) prevents the at least one odorous substance from being supplied when the air that is conducted through the air duct (8) is below a lower temperature, for example -20°C, and/or when the air that is conducted through the air duct is above an upper temperature, for example +60°C.

3. The device according to claim 1 or 2, **characterised in that** the closing device (5) is a flap or a damper which alternately completely opens or closes an opening to the odorous substance carrier (2), or is a flap or a damper which variably at least partially opens or closes the opening to the odorous substance carrier (2).

4. A method for supplying at least one odorous substance into the passenger compartment of a motor vehicle, comprising the following steps:
- conducting air through an air duct (8),
- supplying at least one odorous substance into the air that is conducted through the air duct (8),
- controlling or regulating the amount of the at least one odorous substance that is supplied as a function of the time, and
- introducing the air that has been enriched with the at least one odorous substance into the passenger compartment of the motor vehicle,
wherein the amount of the at least one odorous substance that is supplied is controlled or regulated as a function of the temperature of the air that is conducted through the air duct (8), **characterised in that** the odorous substance carrier (2) is disposed in the air duct (8) and the at least one temperature sensor (3) is disposed in and/or on the air duct (8), wherein, as a function of the time, the preferably variable amount of the at least one odorous substance that is supplied can be controlled by way of curves or algorithms stored in a control unit as a function of the temperature to the effect that a constant or variable amount of the at least one odorous substance is supplied as a function of the time.

5. The method according to claim 4, **characterised in that** no air is conducted through the air duct (8) and/or the supply of the at least one odorous substance into the air that is conducted through the air duct (8) is suppressed when the air that is conducted through the air duct (8) is above an upper temperature, for example +60°C, or when the air that is conducted through the air duct (8) is below a lower temperature, for example -20°C.

6. The method according to claim 4 or 5, **characterised in that** no air is conducted through the air duct (8) and the supply of the at least one odorous substance into the air is suppressed above a first upper passenger compartment temperature, for example +45°C, or below a first lower passenger compartment temperature, for example 0°C, wherein the first upper passenger compartment temperature is higher than the first lower passenger compartment temperature.

7. The method according to claim 6, **characterised in that** air is conducted through the air duct (8) and the supply of the at least one odorous substance into the air is suppressed above a second upper passenger compartment temperature, for example +35°C, or below a second lower passenger compartment temperature, for example 10°C, wherein the second upper passenger compartment temperature is higher than the second lower passenger compartment temperature.

8. The method according to claim 7, **characterised in that** the first upper passenger compartment temperature is higher than the second upper passenger compartment temperature and/or the first lower passenger compartment temperature is lower than the second lower passenger compartment temperature.

9. The method according to claim 7 or 8, **characterised in that** air is conducted through the air duct (8) and at least one odorous substance is supplied to the air that is conducted through the air duct (8) in a sub-range of the interior compartment temperature between the second upper passenger compartment temperature and the second lower passenger compartment temperature, for example between 15°C and 35°C.

10. A computer program product with program code means stored on a computer-readable data medium in order to carry out a method according to one or more of claims 4 to 9 if the computer program is executed on a computer or a corresponding processor.

## Revendications

1. Dispositif (1) servant à l'introduction d'au moins une substance parfumée, dans l'habitacle d'un véhicule automobile, ledit dispositif comprenant :
- un diffuseur de substances parfumées (2) comportant au moins une substance parfumée,
- un conduit d'air (8) servant à l'introduction d'air à enrichir jusqu'au diffuseur de substances parfumées (2) et/ou servant au recyclage, dans l'habitacle du véhicule automobile, d'air enrichi provenant du diffuseur de substances parfumées (2), où la substance parfumée au moins au nombre de un peut être introduite dans le conduit d'air (8),
- un pulseur (4) servant à faire circuler l'air dans le conduit d'air (8) et
- un dispositif de fermeture (5) servant à la commande ou à la régulation de la quantité de la substance parfumée au moins au nombre de un pouvant être introduite par le diffuseur de substances parfumés (2) en fonction du facteur temps,
où la température de l'air à enrichir et/ou enrichi par la substance parfumée au moins au nombre de un, ledit air se trouvant dans le conduit d'air (8), peut être mesurée au moyen au moins d'un capteur de température (3), et le dispositif de fermeture (5) peut être commandé ou régulé en fonction de la température mesurée, **caractérisé en ce que** le diffuseur de substances parfumées (2) est disposé dans le conduit d'air (8), et le capteur de température (3) au moins au nombre de un est disposé dans et/ou sur le conduit d'air (8), où le dispositif (1) comprend une unité de commande, et le dispositif de fermeture (5), au moyen de courbes ou d'algorithmes mémorisés dans l'unité de commande, peut être commandé en fonction de la température, par le fait qu'une quantité - constante ou variable - de la substance parfumée au moins au nombre de un est introduite en fonction du facteur temps.

2. Dispositif selon la revendication 1, **caractérisé en ce qu'**au-dessous d'une température inférieure de l'air dirigé à travers le conduit d'air (8), par exemple -20°C, et/ou au-dessus d'une température supérieure de l'air dirigé à travers le conduit d'air, par exemple +60°C, le pulseur (4) est coupé et/ou le dispositif de fermeture (5) empêche l'introduction de la substance parfumée au moins au nombre de un.

3. Dispositif selon la revendication 1 ou 2, **caractérisé en ce que** le dispositif de fermeture (5) est une tirette ou un volet qui, alternativement, ouvre ou ferme complètement une ouverture placée sur le diffuseur de substances parfumées (2), ou bien est une tirette ou un volet qui ouvre ou ferme au moins partiellement, de façon variable, l'ouverture placée sur le diffuseur de substances parfumées (2).

4. Procédé permettant l'introduction d'au moins une substance parfumée, dans l'habitacle d'un véhicule automobile, ledit procédé comprenant les étapes consistant :
- à diriger de l'air à travers un conduit d'air (8),
- à introduire au moins une substance parfumée, dans l'air dirigé à travers le conduit d'air (8),
- à commander ou à réguler, en fonction du facteur temps, la quantité de la substance parfumée au moins au nombre de un ayant été introduite, et
- à introduire, dans l'habitacle du véhicule automobile, l'air enrichi par la substance parfumée au moins au nombre de un,
où la quantité de la substance parfumée au moins au nombre de un ayant été introduite est commandée ou régulée en fonction de la température de l'air dirigé à travers le conduit d'air (8), **caractérisé en ce que** le diffuseur de substances parfumées (2) est disposé dans le conduit d'air (8), et le capteur de température (3) au moins au nombre de un est disposé dans et/ou sur le conduit d'air (8) où, en fonction du facteur temps, la quantité - de préférence variable - de la substance parfumée au moins au nombre de un ayant été introduite peut, en fonction de la température, être commandée au moyen de courbes ou d'algorithmes mémorisés dans une unité de commande, par le fait qu'une quantité - constante ou variable - de la substance parfumée au moins au nombre de un est introduite en fonction du facteur temps.

5. Procédé selon la revendication 4, **caractérisé en ce qu'**au-dessus d'une température supérieure de l'air dirigé à travers le conduit d'air (8), par exemple +60°C, ou bien au-dessous d'une température inférieure de l'air dirigé à travers le conduit d'air (8), par exemple -20°C, aucune quantité d'air n'est dirigée à travers le conduit d'air (8) et/ou l'introduction de la substance parfumée au moins au nombre de un, dans l'air dirigé à travers le conduit d'air (8), est arrêtée.

6. Procédé selon la revendication 4 ou 5, **caractérisé en ce qu'**au-dessus d'une première température supérieure de l'habitacle, par exemple +45°C, ou bien au-dessous d'une première température inférieure de l'habitacle, par exemple 0°C, aucune quantité d'air n'est dirigée à travers le conduit d'air (8), et l'introduction, dans l'air, de la substance parfumée au moins au nombre de un est arrêtée, où la première température supérieure de l'habitacle est supérieure à la première température inférieure de l'habitacle.

7. Procédé selon la revendication 6, **caractérisé en ce qu'**au-dessus d'une deuxième température supérieure de l'habitacle, par exemple +35°C, ou bien au-dessous d'une deuxième température inférieure de l'habitacle, par exemple 10°C, de l'air est dirigé à travers le conduit d'air (8), et l'introduction, dans l'air, de la substance parfumée au moins au nombre de un est arrêtée, où la deuxième température supérieure de l'habitacle est supérieure à la deuxième température inférieure de l'habitacle.

8. Procédé selon la revendication 7, **caractérisé en ce que** la première température supérieure de l'habitacle est supérieure à la deuxième température supérieure de l'habitacle et/ou la première température inférieure de l'habitacle est inférieure à la deuxième température inférieure de l'habitacle.

9. Procédé selon la revendication 7 ou 8, **caractérisé en ce que,** dans une plage partielle de la température de l'habitacle comprise entre la deuxième température supérieure de l'habitacle et la deuxième température inférieure de l'habitacle, par exemple entre 15°C et 35°C, de l'air est dirigé à travers le conduit d'air (8), et au moins une substance parfumée est introduite dans l'air dirigé à travers le conduit d'air (8).

10. Produit de programme informatique comportant des moyens de programmation qui sont mémorisés sur un support de données pouvant être lu par l'ordinateur, pour mettre en œuvre un procédé selon l'une quelconque ou plusieurs des revendications 4 à 9, quand le programme informatique est mis en œuvre sur un ordinateur ou sur une unité de calcul correspondante.
